# EUROPEAN PATENT APPLICATION

(11) **EP 4 497 395 A1**
(43) Date of publication of application: **29.01.2025**
(21) Application number: 22935800.7
(22) Date of filing: 29.03.2022
(51) Int. Cl.: A61B 10/02, A61B 17/34, A61B 90/00

(54) **BIOPSY NEEDLE MODULE USING MEDICAL IMAGING EQUIPMENT FOR REAL-TIME BIOPSY**

(30) Priority: 28.03.2022 KR 20220038234
(71) Applicant: Cure-in Inc., Goyang-si, Gyeonggi-do 10407 (KR); National Cancer Center, Goyang-si, Gyeonggi-do 10408 (KR)
(72) Inventor: JO, Yung Ho, Goyang-si, Gyeonggi-do 10222 (KR); LEE, Sun Ok, Paju-si, Gyeonggi-do 10893 (KR); PARK, Byoeng Jun, Goyang-si, Gyeonggi-do 10417 (KR); KIM, Yun Jae, Goyang-si, Gyeonggi-do 10391 (KR); KANG, Han Sung, Goyang-si, Gyeonggi-do 10416 (KR)
(74) Representative: RGTH
(86) International application number: PCT/KR2022/004398
(87) International publication number: WO 2023/191124

(57) **Abstract**

The present invention relates to a biopsy needle module using medical imaging equipment, the biopsy needle module being inserted into a biopsy area of a patient to do a biopsy. The biopsy needle module comprises: a needle part (110), made of high-strength and super-elastic material, having overlapping inner needle (113) and outer needle (111); a curved needle frame (120) curving in a perpendicular direction and having, in the front, a needle front end-exposing hole (123a) through which the front end of the needle part (110) is exposed to the outside, and, in the rear, a curved rail (125) that is open along the curved surface; and a needle guide (130), coupled so as to slide along the curved rail (125) of the curved needle frame (120), for supporting, in a straight line, the back end of the needle part (110) the front end of which is inserted in the needle front end-exposing hole (123a), and guiding the needle part (110), in a curved state, in moving forward and backward along the curved needle frame (120).

## Description

### [Technical Field]

The present invention relates to a biopsy needle module, and more particularly to a biopsy needle module that is inserted into a biopsy site of a patient to perform a biopsy procedure while checking an image of the biopsy site of the patient in real time using medical imaging equipment.

### [Background Art]

A biopsy is a type of examination method in which a hollow needle is inserted into an organ in vivo without making an incision in the skin to collect a part of the tissue for pathological histological examination. A biopsy is a preoperative examination of a suspected tumor by sonography, computed tomography (CT), magnetic resonance imaging (MRI), etc. in the initial diagnosis of cancer, and a biopsy is taken based on images.

When taking a biopsy using medical images such as CT or MR, the space in a gantry of medical imaging equipment where a patient is located is usually narrow, and a general biopsy needle is straight and long, taking up a lot of space, whereby it is difficult to introduce the needle into the patient's tissue in the small gantry of the medical imaging equipment.

In order to solve this problem, a biopsy robot system that performs a biopsy procedure on a patient while checking an image in real time in a gantry using a bendable needle device is disclosed in Korean patent Application Publication No. 10-2016-0127653.

In the disclosed "bendable needle device and real-time biopsy robotic system using the same," a robotic end effector moves the bendable needle device to the side of a bed where a patient is prone or lying down using a robot controller such that an operator can insert a needle device into a biopsy site and biopsy a tissue sample in the gantry while checking an image.

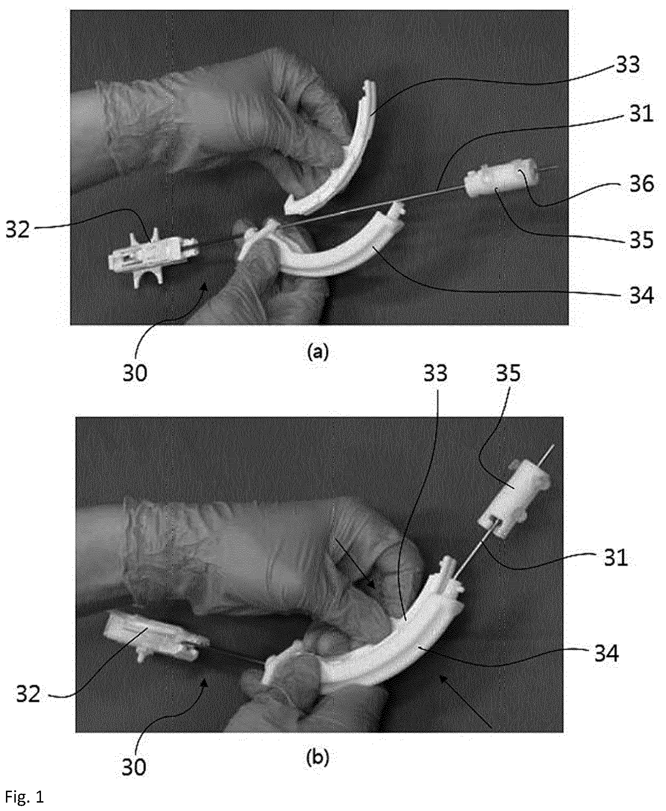

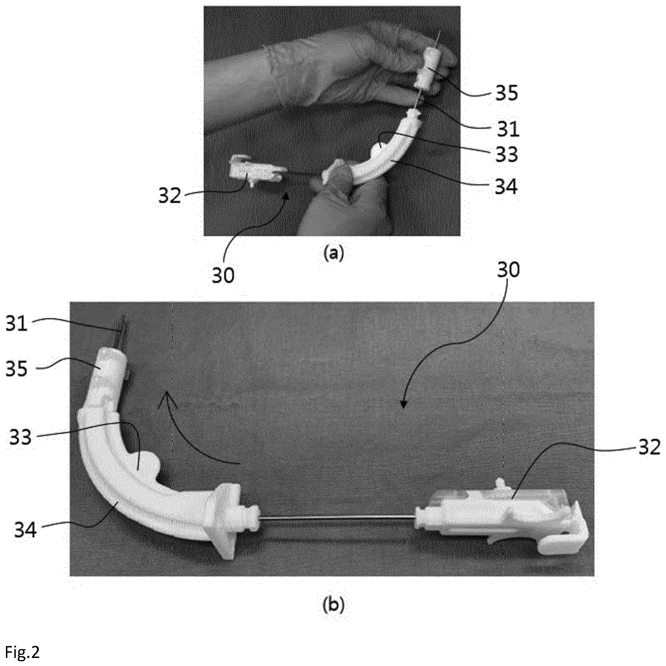

FIGs. 1 and 2 are illustrative views showing a process of assembling a biopsy needle device 30 for use in a conventional real-time biopsy robotic system.

The biopsy needle device 30 must be inserted into a biopsy site of a patient in a curved state from the side of the bed on which the patient is lying. Accordingly, a straight needle 31 is curved and inserted into the biopsy site.

The needle 31 used in the biopsy needle device 30 is made of a highly rigid superelastic material that has superelasticity when being bent and recovers rigidity in a straight part, whereby it is possible for the needle to be bent and then move in a straight line again. However, since deformation may occur if the needle is kept in a bent state for a long time, the needle 31 is stored in a straight form when not in use, and when used for biopsy, the needle 31 is bent by an operator in the field.

To this end, as shown in (a) of FIG. 1, the conventional biopsy needle device 30 includes a needle 31, a biopsy gun body 32 coupled to the rear of the needle 31 to manipulate an inner needle unit and an outer needle unit of the needle 31 so as to move in the biopsy procedure, a guide outer 33 and a guide inner 34 disposed on the path of the needle 31, and a straightener 35 coupled to a front end of the needle 31, the straightener having a marker 36, configured to indicate the current position of the needle 31 on an image, coupled thereto.

When in storage, the needle 31 is stored in a straight state, and the guide outer 33 and the guide inner 34 are stored separately from the needle 31.

For use of the biopsy needle device 30, the operator disposes the guide outer 33 and the guide inner 34 on the path of the needle 31 and assembles them by snapping them together, as shown in (b) of FIG. 1. The assembly of the guide outer 33 and the guide inner 34 causes the path of the needle 31 received therein to be curved.

Subsequently, the assembly is completed by fitting the straightener 35 on the front end of the needle 31, as shown in (a) of FIG. 2, and locating the straightener 35 in contact with the guide outer 33 and the guide inner 34, as shown in (b) of FIG. 2.

The assembled biopsy needle device 30 is then coupled to a robot end effector to perform a biopsy procedure.

However, the conventional biopsy needle device require a significant amount of time for the operator to perform the assembly process, including the assembly of the guide inner and guide outer and the insertion of the straightener, before use.

### [Disclosure]

### [Technical Problem]

The present invention has been made in view of the above problems, and it is an object of the present invention to provide a biopsy needle module that is easy to assemble prior to a procedure.

It is another object of the present invention to provide a biopsy needle module configured such that a manipulation means configured to manipulate a needle unit is separated, whereby the biopsy needle module has a simple configuration.

The above objects and various advantages of the present invention will become apparent to those skilled in the art from a preferred embodiment of the present invention.

### [Technical Solution]

The above objects of the present invention may be accomplished by a biopsy needle module that is inserted into a biopsy site of a patient using medical imaging equipment to perform a biopsy procedure. The biopsy needle module according to the present invention includes a needle unit (110) including an inner needle (113) and an outer needle (111) disposed so as to overlap each other, the needle unit being made of a highly rigid superelastic material, a needle curving frame (120) formed so as to be curved in a vertical direction, the needle curving frame being provided at the front thereof with a needle front end exposure hole (123a) configured to expose a front end of the needle unit (110) to the outside, the needle curving frame being provided at the rear thereof with a curved rail (125) formed so as to be open along a curved surface, and a needle guide (130) slidably coupled along the curved rail (125) of the needle curving frame (120), the needle guide being configured to support a rear end of the needle unit (110) having the front end inserted into the needle front end exposure hole (123a) in a straight shape and to guide the needle unit (110) so as to be moved forward and backward in a curved state along the needle curving frame (120).

In an embodiment, the biopsy needle module may include an inner needle fixing rib (114) fixedly coupled to a rear end of the inner needle (113), the inner needle fixing rib being provided at an upper part thereof with a rear guide coupling protrusion (114a) movably coupled to the needle guide (130), and an outer needle fixing rib (112) fixedly coupled to a rear end of the outer needle (111) in front of the inner needle fixing rib (114), the outer needle fixing rib being provided at an upper part thereof with a front guide coupling protrusion (112a) movably coupled to the needle guide (130), wherein the inner needle fixing rib (114) and the outer needle fixing rib (112) may be coupled to an end effector of a biopsy needle manipulation robotic system to receive information as to whether the inner needle (113) and the outer needle (111) have been moved from the end effector.

In an embodiment, straighteners (124), in which markers (124a) configured to indicate the position of a front end of the needle are received, may be integrally coupled to both sides of a front end of the needle curving frame (120), in which the needle front end exposure hole (123a) is formed.

In an embodiment, the curved rail (125) may be provided with a guide insertion channel (125a) formed so as to be depressed inward along a curved path, the guide insertion channel being configured to allow the needle guide (130) to be moved in a state of being inserted thereinto, a pair of protrusion guide slits (125b) may be formed in both sides of a plate surface of the curved rail (125) in which the guide insertion channel (125a) is formed, the needle guide (130) may be provided at the front thereof with a rail insertion end (133) inserted into the guide insertion channel (125a), and a pair of guide protrusions (135) inserted into the protrusion guide slits (125b) may be formed on both sides of the rail insertion end (133) so as to protrude therefrom, the guide protrusions being configured to support the rail insertion end (133) so as to be moved along the guide insertion channel (125a).

### [Advantageous Effects]

The biopsy needle module according to the present invention has the advantage that the needle unit, the needle curving frame, and the needle guide are stored in an integrally coupled state such that the operator can complete assembly by pressing only the needle guide and sliding the same downward prior to the biopsy procedure, whereby it is possible to significantly reduce the time required for assembly.

In addition, since the straighteners are integrally coupled the needle curving frame, it is possible to reduce the time required to couple the straighteners, unlike the conventional case.

In addition, the biopsy needle module according to the present invention has an improved structure in which the biopsy gun body, which is integrally coupled to the conventional biopsy needle device, is omitted and the inner needle and the outer needle of the needle unit are manipulated using the end effector. Consequently, the present invention has the advantage of enabling more precise manipulation of the inner needle and the outer needle.

### [Description of Drawings]

FIGs. 1 and 2 are illustrative views showing a process of assembling a conventional biopsy needle device before a procedure;
FIG. 3 is an illustrative view showing a biopsy process of a breast biopsy system to which a biopsy needle module according to the present invention is coupled;
FIG. 4 is a perspective view showing a state of the biopsy needle module according to the present invention before assembly;
FIG. 5 is a perspective view showing a state of the biopsy needle module according to the present invention after assembly;
FIG. 6 is an exploded perspective view showing components of the biopsy needle module according to the present invention;
FIG. 7 is a sectional illustrative view showing steps of a process of assembling the biopsy needle module according to the present invention;
FIG. 8 is an illustrative view showing a process in which the biopsy needle module according to the present invention is coupled to an end effector;
FIG. 9 is an illustrative view showing a step in which the biopsy needle module according to the present invention is operated by the end effector; and
FIG. 10 is an illustrative view showing a step in which the biopsy needle module according to the present invention is used to perform a biopsy.

### [Best Mode]

In order to fully understand the present invention, a preferred embodiment of the present invention will be described with reference to the accompanying drawings. The embodiment of the present invention may be changed in various forms, and the scope of the present invention must not be interpreted as being limited to the following embodiment described below in detail. The present embodiment is provided to more completely describe the present invention to a person having ordinary skill in the art to which the present invention pertains. Consequently, the shapes, etc. of elements in the drawings may be exaggerated for clearer description. It should be noted that identical members may be denoted by the same reference numerals in the drawings. A detailed description of related known functions and constructions will be omitted when the same may obscure the subject matter of the present invention.

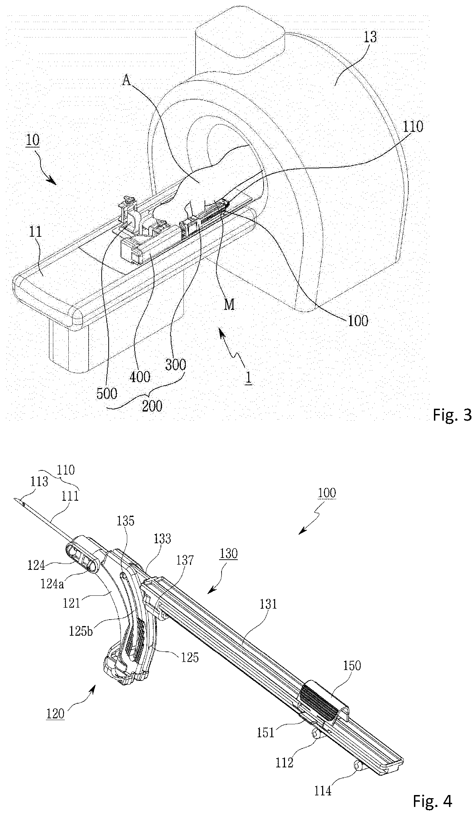

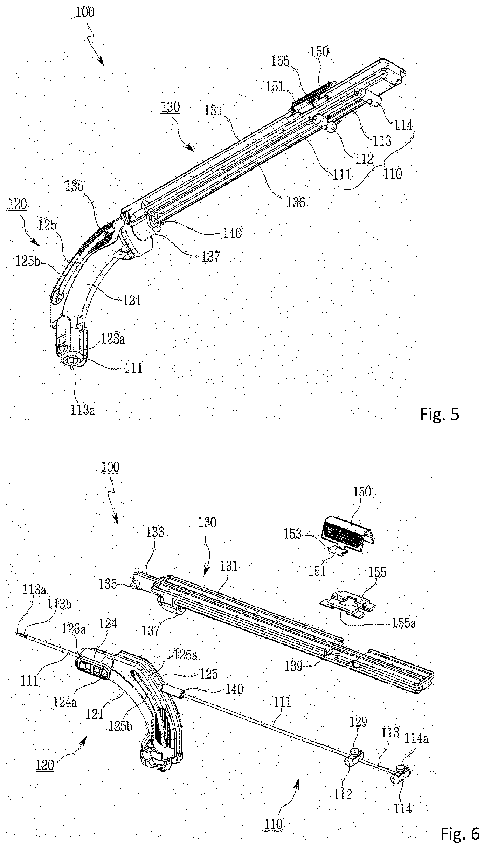

FIG. 3 is an illustrative view showing a biopsy system 1 that performs a biopsy procedure using a biopsy needle module 100 according to the present invention, FIG. 4 is a perspective view showing a state of the biopsy needle module 100 before assembly, FIG. 5 is a perspective view showing a state of the biopsy needle module 100 after assembly, and FIG. 6 is an exploded perspective view showing components of the biopsy needle module 100.

As shown in FIG. 3, the biopsy needle module 100 according to the present invention is applied to the biopsy system 1 and used for a real-time biopsy procedure using medical imaging equipment.

The biopsy system 1 is coupled to medical imaging equipment, such as an MRI, and is inserted into a biopsy site of a patient who is prone or lying on a bed for the medical imaging equipment or a patient who is sitting or standing on upright imaging equipment, such that an operator can accurately biopsy a biopsy sample by inserting a needle into the position of a biopsy target while viewing an image in real time.

The biopsy system 1 includes a biopsy needle module 100 that is inserted into a biopsy site of a patient and a biopsy needle manipulation robotic apparatus 200 that adjusts the position of the biopsy needle module 100 such that the biopsy needle module is inserted into a biopsy target site. The biopsy needle manipulation robotic apparatus 200 includes an end effector 300 to which the biopsy needle module 100 is removably coupled and which manipulates the biopsy needle module 100 and a position adjustment robot 400 that adjusts the position of the end effector 300 such that the biopsy needle module 100 can be inserted into various biopsy target sites.

The biopsy needle manipulation robotic apparatus 200 is provided in a straight line oriented parallel to the patient at a side surface of the bed 11 on which the patient is prone or lying. The biopsy needle module 100 is coupled to the end effector 300 of the biopsy needle manipulation robotic apparatus 200 such that a curved needle unit 110 is inserted into the biopsy location of the lying patient. This allows the needle unit 110 to be inserted into the biopsy site of the patient in a confined space between the bed 11 and an inner wall surface of a gantry 13 to perform a biopsy procedure.

In some cases, if the medical imaging equipment is to be operated in the state in which the patient is in an upright position, the biopsy needle manipulation robotic apparatus 200 may be provided in a direction perpendicular to the side of the upright patient and parallel to the patient.

In the biopsy needle module 100 of the present invention, the needle unit 110 may be stored in a straight shape when not in use, as shown in FIG. 4, and the needle unit 110 is assembled in a curved shape prior to a biopsy procedure, as shown in FIG. 5.

Here, as shown in FIG. 6, the biopsy needle module 100 according to the present invention includes a needle unit 110, a needle curving frame 120 coupled to the needle unit 110 with a front end of the needle unit 110 inserted therein, the needle curving frame having a curved shape, and a needle guide 130 coupled to an upper part of the needle curving frame 120 so as to be movable along the curved shape of the needle curving frame 120, the needle guide being configured to support the needle unit 110.

In the biopsy needle module 100 according to the present invention, the needle curving frame 120 and the needle guide 130 may be stored in a state of being coupled to each other along the path of the needle unit 110, and prior to the biopsy procedure, the operator may move only the needle guide 130 along the needle curving frame 120 to curve the needle unit 110. Accordingly, the assembly time may be significantly reduced when compared to the conventional biopsy needle device 30 shown in (a) of FIG. 1. In addition, the biopsy needle module 100 has the advantage that the construction of the conventional biopsy gun body 32 is omitted and the needle unit 110 is manipulated by the end effector 300, whereby the precision of the operation may be further improved.

The needle unit 110 is manipulated by the end effector 300 and is inserted into a biopsy target site T to biopsy a tissue sample S. As shown in FIG. 6, the needle unit 110 is configured such that the inner needle 113 and the outer needle 111 overlap each other. The inner needle 113 is formed with a larger length than the outer needle 111.

An outer needle fixing rib 112 coupled to the end effector 300 is fixedly coupled to the rear of the outer needle 111, and a front guide coupling protrusion 112a coupled to the needle guide 130 is formed on an upper part of the outer needle fixing rib 112 so as to protrude therefrom.

A sharp tip 113a is provided at the front end of the inner needle 113, and a biopsy recess 113b depressed from the plate surface to biopsy the tissue sample is formed at the rear of the tip 113a. The rear of the inner needle 113 is exposed to the outer side of the outer needle 111 by a predetermined length and is coupled to an inner needle fixing rib 114. The inner needle fixing rib 114 is coupled to the end effector 300, and a rear guide coupling protrusion 114a is formed on an upper part of the inner needle fixing rib 114 so as to protrude therefrom.

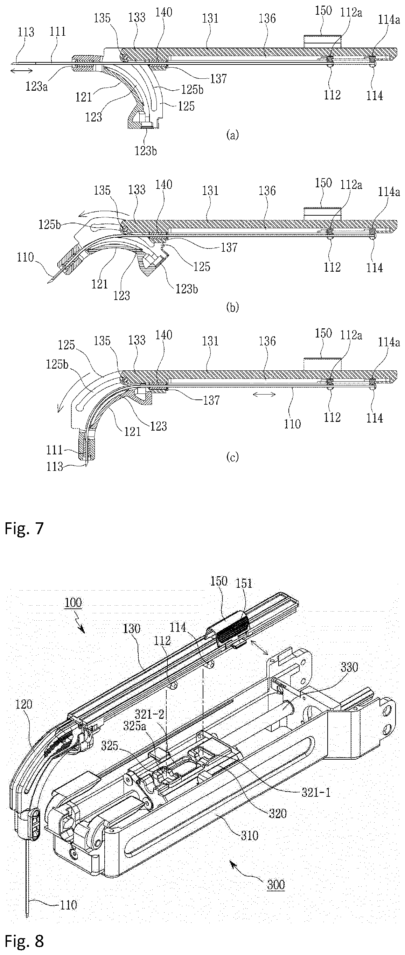

FIG. 8 is an illustrative view showing a state in which the biopsy needle module 100 is mounted to the end effector 300. As shown, the outer needle fixing rib 112 and the inner needle fixing rib 114 are inserted into an inner needle rib insertion recess 321-1 of a trigger module 320 of the end effector 300 and an outer needle rib insertion recess 325a of an outer needle carrier 325, respectively, to allow the biopsy needle module 100 to be mounted to the end effector 300.

The inner needle fixing rib 114 is inserted into the inner needle rib insertion recess 321-1 and is moved with the trigger module 320 when the trigger module is moved forward and backward by a module transfer driving unit 330, and supports the inner needle 113 so as to move forward or backward.

The outer needle fixing rib 112 is inserted into the outer needle rib insertion recess 325a and is moved forward and backward by the module transfer driving unit 330 and an outer needle driving unit 340, and supports the outer needle 111 so as to move forward or backward.

As shown in (c) of FIG. 7, the front guide coupling protrusion 112a and the rear guide coupling protrusion 114a are fitted into and coupled to a coupling protrusion movement rail 136 formed at a lower part of the needle guide 130, are moved together along the coupling protrusion movement rail 136 when the outer needle fixing rib 112 and the inner needle fixing rib 114 are moved forward and backward by the end effector 300, and movably support the needle unit 110.

As shown in (a) of FIG. 7, a front end of the needle unit 110 is exposed toward the front in a state of being coupled to a front end of the needle curving frame 120, a rear end of the needle unit is fixed to the needle guide 130 so as to be movable by the front guide coupling protrusion 112a and the rear guide coupling protrusion 114a in a state of being coupled to the inner needle fixing rib 114 and the outer needle fixing rib 112, and a middle path of the needle unit is inserted into a needle guide tube 137 such that forward and backward movement of the needle unit is guided.

The needle curving frame 120 guides the needle unit 110 and the needle guide 130 such that the needle unit 110, which is held straight by the needle guide 130, is curved before being inserted into the biopsy site.

FIG. 7 is a sectional illustrative view showing a process in which the biopsy needle module 100 is assembled before a procedure. As shown in FIGs. 6 and 7, the needle curving frame 120 includes a frame body 121 having a shape curved at an angle of 90 degrees, a needle receiving channel 123 formed in the frame body 121 to receive the curved needle unit 110, straighteners 124 provided on both sides of a front end of the frame body 121 to receive markers 124a, and a curved rail 125 formed at the rear of the frame body 121 so as to be open along a curved path.

The frame body 121 has a shape that corresponds to the shape in which the needle unit 110 is to be curved for the biopsy procedure on the patient and guides the needle unit 110 and the needle guide 130 so as to be curved together. The needle receiving channel 123 is formed in the frame body 121, as shown in (a) of FIG. 7. A needle front end exposure hole 123a that exposes the needle unit 110 toward the front is formed in the front end of the needle receiving channel 123, and a needle rear end insertion hole 123b that inserts the needle unit 110 into the needle receiving channel 123 is formed in a rear end of the needle receiving channel.

As shown in FIG. 4, the straighteners 124 are provided on both sides of the front end of the frame body 121 to receive the markers 124a therein. The biopsy needle module 100 according to the present invention may be fixedly coupled to both sides of the needle curving frame 120 without the need for separate straighteners 124, eliminating conventional cumbersomeness of the operator performing insertion through the needle unit 110.

The curved rail 125 is formed at the rear of the frame body 121 so as to be open along the curved path to guide the needle guide 130 and a needle 31 so as to be moved in a curved state while sliding. A guide insertion channel 125a, into which a rail insertion end 133 of the needle guide 130 is inserted, is formed between the curved rails 125.

As shown in (a) of FIG. 7, in a state before assembly, the guide insertion channel 125a is formed parallel with the needle front end exposure hole 123a. Accordingly, the rear end of the needle unit 110 may be located horizontally at a lower part of the needle guide 130 in the state in which the front end of the needle unit is coupled to the needle front end exposure hole 123a.

A pair of protrusion guide slits 125b is formed in both sides of a plate surface of the curved rail 125 by incision. Guide protrusions 135 formed on both sides of the rail insertion end 133 of the needle guide 130 are inserted into the protrusion guide slits 125b.

As shown in FIG. 4 and (a) of FIG. 7, the guide protrusions 135 are fitted into the protrusion guide slits 125b to maintain the state in which the needle curving frame 120 and the needle guide 130 are coupled to each other. In addition, as shown in (a) of FIG. 7, (b) of FIG. 7, and (c) of FIG. 7, when the operator sequentially applies force to the needle guide 130 and the needle guide 130 is moved along the curved rail 125, the needle guide 130 is supported so as to be moved along the curved path without deviating from the needle curving frame 120.

In the process, the needle unit 110, which is located in a straight shape at the lower part of the needle guide 130, is guided into the needle receiving channel 123 in the needle curving frame 120 as the needle guide 130 moves, and when the needle guide 130 is fully moved to the opposite side of the curved rail 125, the needle unit 110 is fully received in the needle receiving channel 123, as shown in (c) of FIG. 7.

The needle unit 110 is disposed in a straight shape at the lower part of the needle guide 130, but is curved in the needle curving frame 120 and then restored in a straight shape and moved in the needle front end exposure hole 123a.

The needle guide 130 supports the needle unit 110 and is moved along the curved shape of the needle curving frame 120 to guide the needle unit 110 so as to be curved. In addition, the needle guide 130 is coupled to the end effector 300 to guide the needle unit 110 so as to be inserted into the biopsy location through the needle curving frame 120 by manipulation of the end effector 300.

The needle guide 130 is provided in the form of a bar having a predetermined length, as shown in FIG. 6. The needle guide 130 includes a straight needle guide body 131 that movably supports the needle unit 110, a rail insertion end 133 that protrudes from a front end of the needle guide body 131 and is inserted into the curved rail 125 of the needle curving frame 120, a pair of guide protrusions 135 formed on both sides of the rail insertion end 133, a coupling protrusion movement rail 136 formed so as to be depressed in a bottom surface of the needle guide body 131 in a longitudinal direction to movably support the front guide coupling protrusion 112a and the rear guide coupling protrusion 114a of the needle unit 110, a needle guide tube 137 provided at a lower part of a boundary region between the rail insertion end 133 and the needle guide body 131, the needle guide tube being configured to allow a needle movement tube 140 to be inserted thereinto, and a locking member coupling recess 139 formed in an upper surface of a rear end of the needle guide body 131 by incision, the locking member coupling recess being configured to allow a locking member 150 to be coupled thereto.

The needle guide body 131 has a length capable of covering the entirety of the remaining length of the needle unit 110, the front end of which is inserted into the needle curving frame 120. The needle guide body 131 has a width that allows the needle guide body to be seated on the trigger module 320 when coupled to the end effector 300, as shown in FIG. 8.

The rail insertion end 133 protrudes horizontally forward from the front end of the needle guide body 131 and is inserted into the guide insertion channel 125a of the curved rail 125. At this time, the pair of guide protrusions 135 formed on both sides of the rail insertion end 133 is fitted into the protrusion guide slits 125b to support the rail insertion end 133 so as not to deviate from the guide insertion channel 125a.

The coupling protrusion movement rail 136 is formed so as to be depressed in the bottom surface of the needle guide body 131 in the longitudinal direction, as shown in (a) of FIG. 7. The front guide coupling protrusion 112a and the rear guide coupling protrusion 114a are slidably coupled to the coupling protrusion movement rail 136.

When the inner needle fixing rib 114 and outer needle fixing rib 112 are moved forward and backward by manipulation of the end effector 300, the front guide coupling protrusion 112a and the rear guide coupling protrusion 114a are moved along the coupling protrusion movement rail 136 in conjunction therewith, causing the inner needle 113 and the outer needle 111 to move independently.

As shown in FIG. 6, the needle movement tube 140 is coupled on the path of the needle unit 110 to support smooth movement of the needle unit 110 forward and backward.

The needle guide tube 137 is provided in the lower part of the boundary region between the rail insertion end 133 and the needle guide body 131 to receive the needle movement tube 140 therein. As shown in (c) of FIG. 7, when the needle guide 130 is moved to the rear end of the curved rail 125 to complete assembly for a biopsy procedure, the needle movement tube 140 is received in the needle guide tube 137 to support the needle unit 110 so as to be movable stably in a curved state.

The locking member 150 is coupled to the locking member coupling recess 139 of the needle guide 130, and when the end effector 300 and the biopsy needle module 100 are coupled to each other, the locking member serves to lock the biopsy needle module 100 such that the coupled state of the biopsy needle module is maintained.

The locking member 150 is formed with a plate surface convexly folded upward. The locking member 150 is elastically closed or returned to the initial state thereof when gripped by the operator's hand, and is variable in width.

A pair of locking buttons 151 is provided at both ends of the locking member 150, and the locking buttons 151 are connected to the locking member 150 via a button support arm 153.

The locking buttons 151 are caught by the casing unit 310 of the end effector 300 when the biopsy needle module 100 is coupled to the end effector 300, as shown in FIG. 8. The operator closes the locking member 150 such that the locking buttons 151 are inserted into the casing unit 310, and releases the pressure applied to the locking member 150 such that the locking buttons 151 are fitted into and coupled to button coupling recesses (not shown) formed in an inner wall surface of the casing unit 310.

A guide coupling saddle 155 is provided at lower parts of the locking buttons 151, and the guide coupling saddle 155 is coupled to the locking member coupling recess 139 to support the locking buttons 151 such that only the locking buttons 151 are exposed to both sides of the needle guide 130.

A process of using the biopsy needle module 100 according to the present invention having the above configuration and a biopsy procedure process will be described with reference to FIGs. 3 to 10.

The operator unseals the sealed biopsy needle module 100 and assembles the straight needle unit 110 into a curved shape. Upon unpacking, the needle guide 130 is located above the curved rail 125 of the needle curving frame 120, as shown in FIG. 4.

In this state, the operator grasps the rear end of the needle guide 130 with their hand and presses the needle guide downward. As shown in (a) of FIG. 7, when the needle guide 130 is pressed by the hand, the rail insertion end 133 is moved along the protrusion guide slit 125b of the curved rail 125, and the needle unit 110 is also curved by elasticity, as shown in (b) of FIG. 7.

When the needle guide 130 is fully moved to the lower part of the curved rail 125, as shown in (c) of FIG. 7, the needle guide 130 is located so as to be connected to the needle curving frame 120, a front region of the needle unit 110 is located in the needle receiving channel 123 of the needle curving frame 120 in a curved state, and a rear region of the needle unit 110 is located in the lower part of the needle guide 130 in a straight state.

The operator couples the biopsy needle module 100 thus assembled to the end effector 300 of the biopsy needle manipulation robotic apparatus 200, which is fixedly coupled to the bed 11 of the medical imaging equipment 10, as shown in FIG. 3.

As shown in FIG. 8, the inner needle fixing rib 114 and the outer needle fixing rib 112 of the biopsy needle module 100 are fitted into the inner needle rib insertion recess 321-1 and the outer needle rib insertion recess 325a of the end effector 300, respectively.

The locking buttons 151 of the locking member 150 are fixed to the button coupling recesses (not shown) formed in the upper inner wall surface of the casing unit 310 of the end effector 300.

The inner needle 113 is coupled to the inner needle rib insertion recess 321-1 of the trigger module 320 and is moved forward and backward by the module transfer driving unit 330. In a state of being inserted into the outer needle rib insertion recess 325a of the outer needle carrier 325, the outer needle 111 is moved with the trigger module 320 or is moved forward and backward with the outer needle carrier 325 in the trigger module 320.

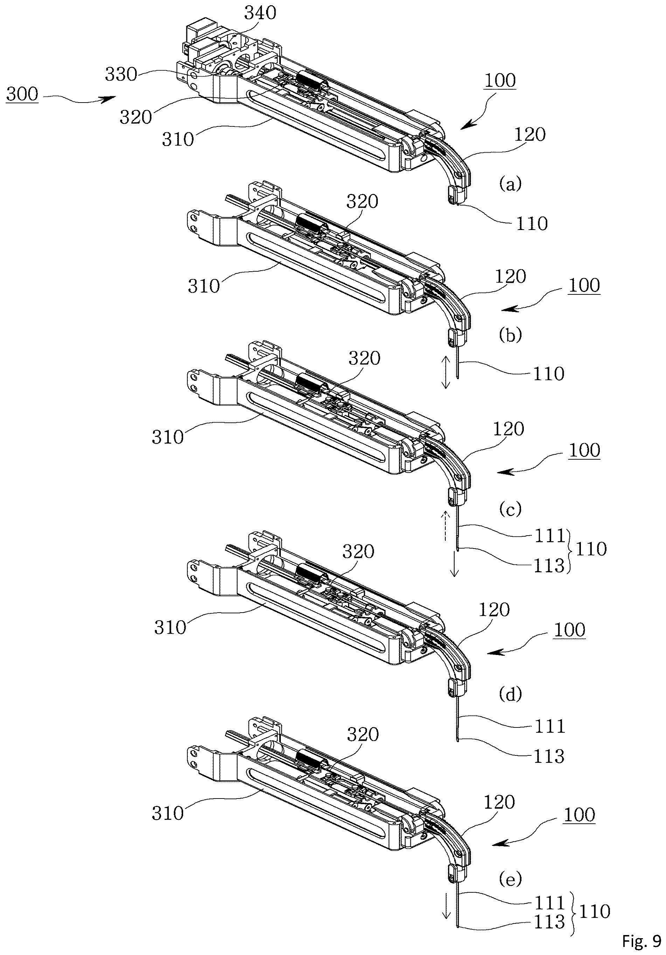

The outer needle carrier 325 is driven by the outer needle driving unit 340 (see (a) of FIG. 9).

When fixing of the biopsy needle module 100 is completed, the needle unit 110 is inserted into the biopsy site M of the patient A, as shown in FIG. 3.

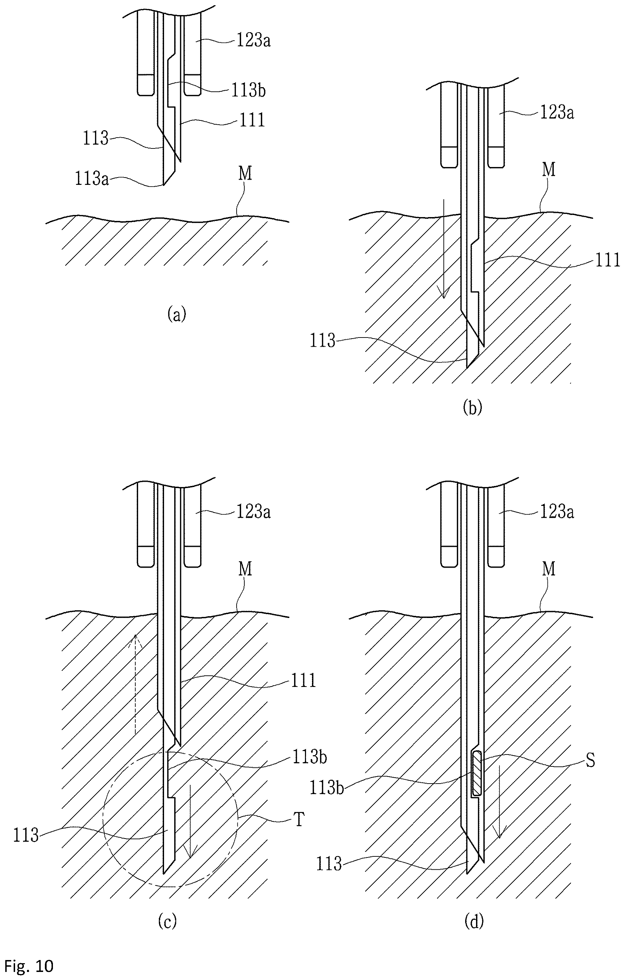

FIG. 9 is an illustrative view showing the position of the needle unit 110 in each step of the biopsy procedure, and FIG. 10 is an illustrative view showing a process in which the needle unit 110 is inserted into the biopsy site M to biopsy the biopsy sample S.

As shown in (a) of FIG. 9 and (a) of FIG. 10, when the biopsy needle module 100 is coupled to the end effector 300, the needle unit 110 is located in the needle curving frame 120 in a received state.

In this state, the operator moves the bed 11 into the gantry 13, manipulates the biopsy needle manipulation robotic apparatus 200 using the navigation unit (not shown) while viewing a captured image, and manipulates the position of the needle unit 110 of the biopsy needle module 100 such that the needle unit is moved to the target biopsy site.

As shown in (b) of FIG. 9 and (b) of FIG. 10, when the module transfer driving unit 330 is driven in a forward direction, the needle unit 110 is moved to the needle curving frame 120 in a curved state. This causes the inner needle 113 and the outer needle 111 to penetrate the biopsy site M.

Subsequently, as shown in (c) and (d) of FIG. 9 and (c) of FIG. 10, the inner needle 113 penetrates the biopsy target position T by the forward driving of the module transfer driving unit 330, and the outer needle 111 is moved backward by the reverse driving of the outer needle driving unit 340.

Subsequently, as shown in (e) of FIG. 9 and (d) of FIG. 10, when the outer needle 111 is shot by the elastic force of the elastic member and is moved forward by the length that the inner needle 113 has been moved forward, a cutting surface formed at the front end of the outer needle 111 cuts the biopsy tissue.

In this process, the tissue sample S is received in the biopsy recess 113b and covered by the outer needle 111, whereby the biopsy procedure may be completed.

After biopsy is completed, the biopsy needle module 100 is moved backward in the reverse direction and is separated from the biopsy site of the patient.

As described above, the biopsy needle module according to the present invention has the advantage that the needle unit, the needle curving frame, and the needle guide are stored in an integrally coupled state such that the operator can complete assembly by pressing only the needle guide and sliding the same downward prior to the biopsy procedure, whereby it is possible to significantly reduce the time required for assembly.

In addition, since the straighteners are integrally coupled the needle curving frame, it is possible to reduce the time required to couple the straighteners, unlike the conventional case.

In addition, the biopsy needle module according to the present invention has an improved structure in which the biopsy gun body, which is integrally coupled to the conventional biopsy needle device, is omitted and the inner needle and the outer needle of the needle unit are manipulated using the end effector. Consequently, the present invention has the advantage of enabling more precise manipulation of the inner needle and the outer needle.

The embodiment of the present invention described above is exemplary only, and a person having ordinary skill in the art to which the present invention pertains will recognize that various modifications and other equivalent embodiments are possible therefrom. It will therefore be well understood that the present invention is not limited to the form recited in the above detailed description. Consequently, the true scope of technical protection of the present invention is to be determined by the technical ideas of the appended claims. In addition, the present invention is to be understood to include all variations, equivalents, and substitutes within the spirit and scope of the present invention as defined by the appended claims.

### [Description of Reference Symbols]

1: Biopsy system 10: Medical imaging equipment
11: Bed 13: Gantry
20: Fixing frame 30: Conventional biopsy needle device
31: Needle 32: Biopsy gun body
33: Guide outer 34: Guide inner
35: Straightener 36: Marker
100: Biopsy needle module 110: Needle unit
111: Outer needle 112: Outer needle fixing rib
112a: Front guide coupling protrusion 113: Inner needle
113a: Tip 113b: Biopsy recess
114: Inner needle fixing rib 114a: Rear guide coupling protrusion
120: Needle curving frame 121: Frame body
123: Needle receiving channel 123a: Needle front end exposure hole
123b: Needle rear end insertion hole 124: Straightener
124a: Marker 125: Curved Rail
125a: Guide insertion channel 125b: Protrusion guide slit
130: Needle guide 131: Needle guide body
133: Rail insertion end 135: Guide protrusion
136: Coupling protrusion movement rail 137: Needle guide tube
139: Locking member coupling recess 140: Needle movement tube
150: Locking member 151: Locking button
153: Button support arm 155: Guide coupling saddle
200: Biopsy needle manipulation robotic apparatus 300: End effector
310: Casing unit 320: Trigger module
321-1: Inner needle rib insertion recess 321-2: Carrier movement channel
325: Outer needle carrier 325a: Outer needle rib insertion recess
330: Module transfer driving unit 340: Outer needle driving unit
400: Position adjustment robot 500: Height adjustment fixing jig
A: Patient
M: Biopsy site
T: Target biopsy position
S: Tissue sample

## Claims

1. A biopsy needle module that is inserted into a biopsy site of a patient using medical imaging equipment to perform a biopsy procedure, the biopsy needle module comprising:
a needle unit (110) comprising an inner needle (113) and an outer needle (111) disposed so as to overlap each other, the needle unit being made of a highly rigid superelastic material;
a needle curving frame (120) formed so as to be curved in a vertical direction, the needle curving frame being provided at a front thereof with a needle front end exposure hole (123a) configured to expose a front end of the needle unit (110) to an outside, the needle curving frame being provided at a rear thereof with a curved rail (125) formed so as to be open along a curved surface; and
a needle guide (130) slidably coupled along the curved rail (125) of the needle curving frame (120), the needle guide being configured to support a rear end of the needle unit (110) having the front end inserted into the needle front end exposure hole (123a) in a straight shape and to guide the needle unit (110) so as to be moved forward and backward in a curved state along the needle curving frame (120).

2. The biopsy needle module according to claim 1, comprising:
an inner needle fixing rib (114) fixedly coupled to a rear end of the inner needle (113), the inner needle fixing rib being provided at an upper part thereof with a rear guide coupling protrusion (114a) movably coupled to the needle guide (130); and
an outer needle fixing rib (112) fixedly coupled to a rear end of the outer needle (111) in front of the inner needle fixing rib (114), the outer needle fixing rib being provided at an upper part thereof with a front guide coupling protrusion (112a) movably coupled to the needle guide (130), wherein
the inner needle fixing rib (114) and the outer needle fixing rib (112) are coupled to an end effector of a biopsy needle manipulation robotic system to receive information as to whether the inner needle (113) and the outer needle (111) have been moved from the end effector.

3. The biopsy needle module according to claim 2, wherein straighteners (124), in which markers (124a) configured to indicate a position of a front end of the needle are received, are integrally coupled to both sides of a front end of the needle curving frame (120), in which the needle front end exposure hole (123a) is formed.

4. The biopsy needle module according to claim 3, wherein
the curved rail (125) is provided with a guide insertion channel (125a) formed so as to be depressed inward along a curved path, the guide insertion channel being configured to allow the needle guide (130) to be moved in a state of being inserted thereinto,
a pair of protrusion guide slits (125b) is formed in both sides of a plate surface of the curved rail (125) in which the guide insertion channel (125a) is formed,
the needle guide (130) is provided at a front thereof with a rail insertion end (133) inserted into the guide insertion channel (125a), and
a pair of guide protrusions (135) inserted into the protrusion guide slits (125b) is formed on both sides of the rail insertion end (133) so as to protrude therefrom, the guide protrusions being configured to support the rail insertion end (133) so as to be moved along the guide insertion channel (125a).
